(19) Europäisches Patentamt
European Patent Office
Office européen des brevets

(11) Numéro de publication : **0 383 655 B1**

(12) **FASCICULE DE BREVET EUROPEEN**

(45) Date de publication du fascicule du brevet : **28.04.93 Bulletin 93/17**

(51) Int. Cl.$^5$ : **A61K 7/42**, A61K 7/48, A61K 7/06

(21) Numéro de dépôt : **90400347.2**

(22) Date de dépôt : **08.02.90**

# (54) Utilisation en cosmétique de diorganopolysiloxanes à fonction dibenzoyleméthane et nouvelles compositions cosmétiques contenant ces composés, destinées à la protection de la peau et des cheveux.

(30) Priorité : **15.02.89 FR 8901991**

(43) Date de publication de la demande : **22.08.90 Bulletin 90/34**

(45) Mention de la délivrance du brevet : **28.04.93 Bulletin 93/17**

(84) Etats contractants désignés : **AT BE CH DE DK ES FR GB GR IT LI NL SE**

(56) Documents cités :
**EP-A- 0 138 590**
**WO-A-88/09663**
**GB-A- 2 185 396**
**US-A- 4 696 969**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Lang, Gérard**
**44 avenue Lacour**
**F-95210 Saint-Gratien (FR)**
Inventeur : **Richard, Hervé**
**48, rue de l'Ermitage**
**F-75020 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 383 655 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à l'utilisation en cosmétique, notamment en tant qu'agents filtrant le rayonnement UV, de diorganopolysiloxanes à fonction dibenzoylméthane ainsi qu'aux nouvelles compositions cosmétiques contenant ces composés, destinées à la protection de la peau et des cheveux.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est donc intéressant de disposer de composés absorbant les rayons UV sur une large bande afin de pouvoir filtrer à la fois les rayons UV-A et UV-B.

On sait par ailleurs que les constituants entrant dans les préparations cosmétiques ne possèdent pas toujours une stabilité suffisante à la lumière et qu'ils se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique, et en particulier dans les huiles et graisses.

Il est également souhaitable d'assurer aux cheveux une bonne protection contre la dégradation photochimique afin d'éviter en particulier une décoloration ou un changement de nuance.

On sait, par ailleurs, greffer sur des chaînes de polymères carbonés synthétiques, de polymères naturels, d'hydrolysats de protéines ou de polyaminoamides, des restes de molécules ayant un effet filtre vis-à-vis du rayonnement UV; ces polymères greffés décrits par exemple dans les brevets français n° 2 197 023, 2 237 912, 2 531 960, 2 548 018, 2 549 069, 2 586 692 et 2 586 693 peuvent être utilisés pour préparer des compositions cosmétiques protectrices de l'épiderme humain ou anti-solaires. On a cependant constaté que ces polymères greffés sont généralement peu solubles dans les solvants cosmétiques usuels, notamment dans les supports gras, et qu'ils forment des films dont la structure est trop rigide.

On connaît également des émulsions de polyorganosiloxanes greffés par des composés benzophénone ou parabenzoate à groupements trialkoxysilane, par polymérisation "in situ" en émulsion, telles que décrites dans le brevet US 4 696 969.

On connaît également des composés silanes ou siloxanes greffés par des groupements méthallylcinnamate, méthallylsalicylate ou méthallylparaminobenzoate filtrant les rayons UV-B d'après la demande EP-A-0 138 590.

Or, la demanderesse a découvert que certains diorganopolysiloxanes à fonction dibenzoylméthane présentaient, de manière étonnante, de bonnes propriétés cosmétiques associées à de bonnes propriétés filtrantes dans une large gamme de longueurs d'onde allant de 280 à 360 nm. Ils présentent notamment un excellent caractère liposoluble, ce qui les rend utilisables dans les supports gras utilisés en cosmétique. Outre leur bon pouvoir filtrant et leur bonne solubilité dans les corps gras et les solvants cosmétiques usuels, ces diorganopolysiloxanes à fonction dibenzoylméthane présentent une excellente stabilité chimique et photochimique et ont l'avantage d'apporter de la douceur à la peau et aux cheveux, par lesquels ils sont bien tolérés.

La présente invention a donc pour objet l'utilisation en cosmétique, en particulier en tant qu'agents filtrant le rayonnement UV de longueurs d'onde comprises entre 280 et 360 nm, de diorganopolysiloxanes à fonction dibenzoylméthane choisis parmi ceux de formule :

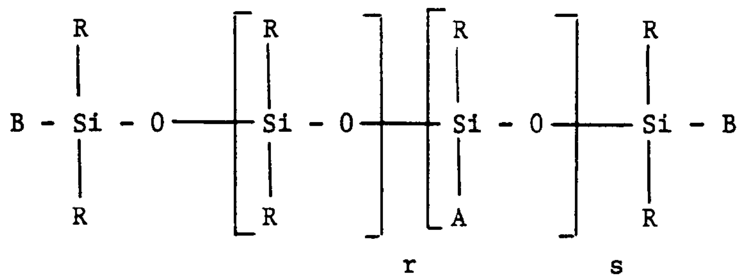

(1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

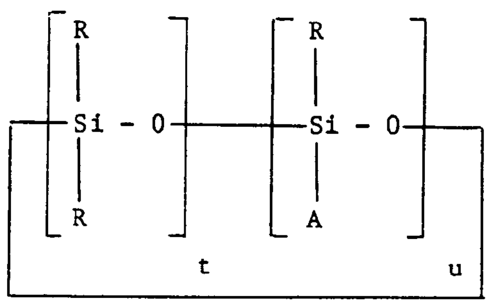

(2)

dans laquelle

R a la même signification qu'à la formule (1),

u est un nombre compris entre 1 et 20 inclus et

t est un nombre compris entre 0 et 20 inclus,

t + u est égal ou supérieur à 3;

formules dans lesquelles le symbole A est un radical de formule :

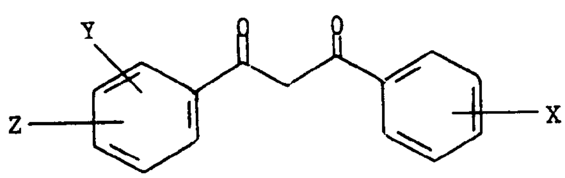

(3)

dans laquelle :

- X est choisi parmi un atome d'hydrogène, un radical alkyle linéaire ou ramifié en $C_1$-$C_8$, un radical alcoxy linéaire ou ramifié en $C_1$-$C_8$,
- Y est choisi parmi un atome d'hydrogène, un groupe hydroxyle et un radical alcoxy linéaire ou ramifié en $C_1$-$C_4$,
- Z est un reste divalent de formule :

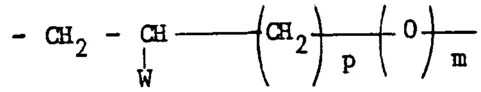

dans lequel m est 0 ou 1, p est un nombre entier compris entre 1 et 10 inclusivement et W est choisi parmi un atome d'hydrogène et un radical alkyle en $C_1$-$C_4$, sous réserve que, lorsque m est égal à 0, Y représente un radical alcoxy ou OH et soit situé en position ortho par rapport à Z.

Parmi les radicaux alkyle linéaires ou ramifiés, on cite plus particulièrement les radicaux méthyle, éthyle, n-propyle, isopropyle, n-butyle, isobutyle, tert.-butyle, n-amyle, isoamyle, néopentyle, n-hexyle, n-heptyle, n-octyle, éthyl-2 hexyle et tert.-octyle.

Parmi les radicaux alcoxy linéaires ou ramifiés, on cite plus particulièrement les radicaux méthoxy, éthoxy, n-propoxy, isopropoxy, n-butoxy, isobutoxy, tert.-butoxy, n-amyloxy, isoamyloxy, néopentyloxy, n-hexyloxy, n-heptyloxy, n-octyloxy, éthyl-2 hexyloxy et tert.-octyloxy.

On préfère plus particulièrement les polymères statistiques ou à blocs de formules (1) et (2) présentant au moins l'une des caractéristiques suivantes :

- R est méthyle,
- B est méthyle,
- r est compris entre 5 et 20 inclus,
- s est compris entre 2 et 15 inclus,
- t + u est compris entre 3 et 10 inclus,
- X est H,
- Y est H, OH ou méthoxy,
- Z est un reste divalent dans lequel m = 0 ou 1, p = 1, W = H ou méthyle.

Pour préparer les polymères de formules (1) et (2), on peut par exemple partir du polymère correspondant dans lequel tous les radicaux A sont des atomes d'hydrogène.

Ce polymère est dénommé par la suite polymère à SiH; les groupes SiH peuvent être présents dans la chaîne et/ou aux extrémités de chaîne. Ces polymères à SiH sont des produits bien connus dans l'industrie des silicones et sont généralement disponibles dans le commerce.

Ils sont par exemple décrits dans les brevets américains US-A-3 220 972, US-A-3 436 366, US-A-3 697 473 et US-A-4 340 709.

Ce polymère à SiH peut donc être choisi parmi ceux de formule :

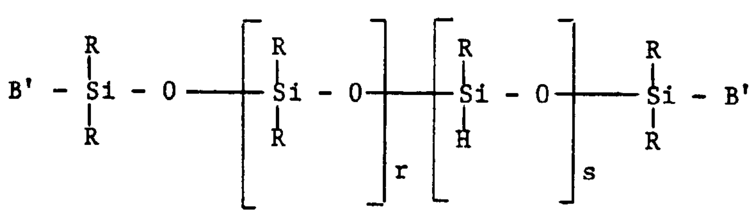

(4)

dans laquelle R, r et s ont la signification donnée ci-dessus pour la formule (1) et les radicaux B', identiques ou différents, sont choisis parmi les radicaux R et un atome d'hydrogène,
et de formule :

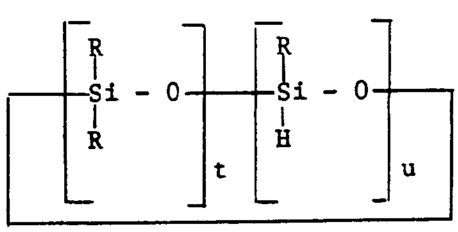

(5)

dans laquelle R, t et u ont la signification donnée ci-dessus pour la formule (2).

Sur ce polymère à SiH de formule (4) ou (5), on effectue une réaction d'hydrosilylation en présence d'une

quantité catalytiquement efficace d'un catalyseur au platine sur un dérivé organique de dibenzoylméthane choisi parmi ceux de formule :

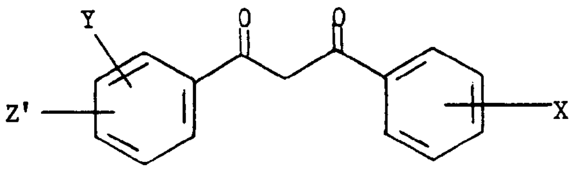

(6)

dans laquelle X et Y ont la signification donnée ci-dessus à la formule (3) et Z' représente le radical insaturé monovalent de formule :

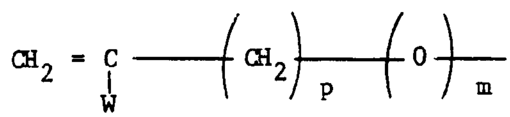

dans laquelle p, m et W ont la signification donnée à la formule (3) ci-dessus sous réserve que lorsque m est égal à 0, Y représente un radical OH ou alcoxy et soit situé en position ortho par rapport à Z'.

Les produits de formule (6) sont, pour la plupart, des produits connus. Ils peuvent être synthétisés notamment suivant les modes opératoires décrits dans les publications référencées dans les résumés des CHEMICAL ABSTRACTS suivants : vol. 58, 11316e, Vol. 93, 8051h, Vol. 101, 191 435g et Vol. 94, 308 660p, ainsi que dans les brevets FR-A-2 506 156, FR-A-2 513 992 et FR-A-2 526 658.

Un procédé convenable pour m = 0 est le suivant :

Au cours d'une première étape a) on fait réagir de l'acétophénone hydroxylée en position 2, 3 ou 4 sur un halogénure d'alcényle de formule :

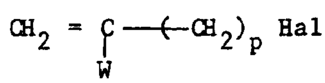

(7)

dans laquelle W et p ont la signification donnée ci-dessus à la formule (6) et Hal représente un atome d'halogène, de préférence le chlore ou le brome, en présence d'une base, par exemple en présence d'un hydroxyde ou carbonate de métal alcalin ou alcalino-terreux ou d'un amidure, alcoolate ou hydrure alcalin, dans un solvant compatible avec la nature de la base tel que l'eau ou un solvant organique tel qu'un alcool, le dioxane, le diméthylsulfoxyde ou le diméthylformamide, à une température comprise entre la température ambiante et le point d'ébullition du solvant, pour obtenir un produit de formule :

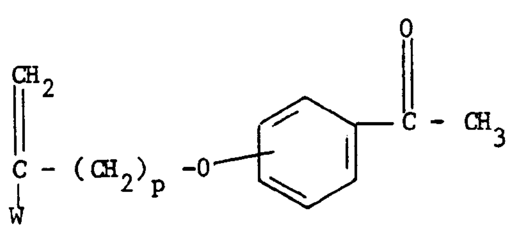

(8)

Au cours d'une deuxième étape b) on effectue un réarrangement de CLAISEN décrit par TARBELL (Organic Réactions, Vol. 2, John WILEY, New-York, 1944, page 1) par chauffage à au moins 170°C environ du composé de formule (8) en présence éventuellement d'un solvant pour obtenir le produit de formule :

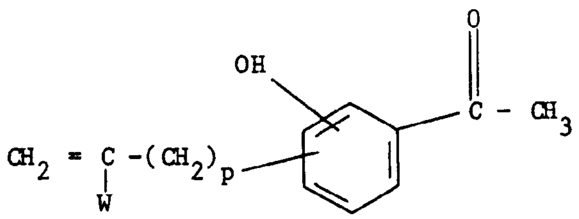

(9)

Au cours d'une troisième étape c) on condense dans la pyridine le produit de formule (9) sur le chlorure de benzoyle de formule :

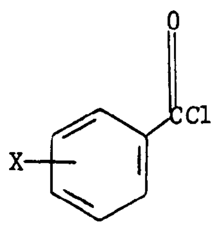

(10)

pour obtenir un produit de formule :

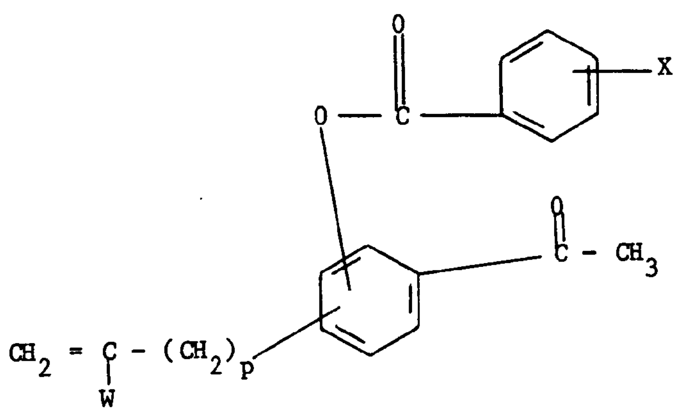

(11)

et on obtient finalement le produit de formule (6) avec m = 0 en effectuant au cours d'une quatrième étape d), en présence de potasse et dans la pyridine un réarrangement de BAKER VENKATARAMAN sur le produit de formule (11).

Les composés de formule (6) dans laquelle m = 0 ou 1 et Y est différent de OH sont obtenus par condensation d'un ester de formule (12) sur une acétophénone de formule (13).

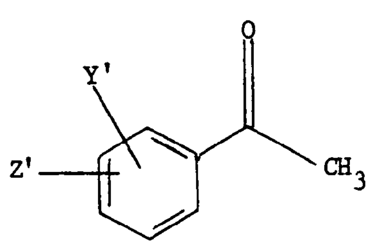

(13)

+

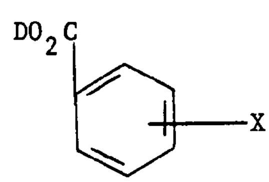

(12)

Dans le composé de formule (12), X a la signification donnée ci-dessus à la formule (3) et D représente un reste alkyle en $C_1$-$C_6$. Dans le composé de formule (13), Z' a la signification donnée ci-dessus à la formule (6) et Y' représente un atome d'hydrogène, ou un radical alcoxy en $C_1$-$C_4$. La réaction est effectuée en présence d'une base, par exemple en présence d'un alcoolate, hydrure ou amidure alcalin, dans un solvant compatible avec la nature de la base tel que le toluène, l'éther isopropylique, le dioxane, le tétrahydrofuranne, le diméthoxy-1,2 éthane, le diméthylsulfoxyde ou le diméthylformamide, à une température comprise entre la température ambiante et le point d'ébullition du solvant.

L'acétophénone de formule (13) peut être préparée selon des méthodes connues.

Par exemple :

- lorsque m = 1 et Y' = H, l'acétophénone est obtenue selon le mode opératoire décrit ci-dessus pour la préparation du composé (8),
- lorsque m = 0 et Y' = alcoxy en $C_1$-$C_4$, l'acétophénone est obtenue par alkylation du composé de formule (9) au moyen d'un halogénure ou d'un sulfate d'alkyle en $C_1$-$C_4$ selon le mode opératoire décrit à l'étape a) ci-dessus.

Les catalyseurs au platine utilisés pour réaliser la réaction d'hydrosilylation des polymères de formules (4) et (5) sur le dérivé organique de formule (6) sont amplement décrits dans la littérature. On peut en particulier citer les complexes du platine et d'un produit organique décrit dans les brevets américains US-A- 3 159 601, US-A-3 159 602, US-A-3 220 972 et les brevets européens EP-A-57 459, EP-A-188 978 et EP-A-190 530 et les complexes du platine et d'organopolysiloxane vinylé décrits dans les brevets américains US-A-3 419 593, US-A-3 377 432 et US-A-3 814 730.

Pour faire réagir le polymère à SiH de formule (4) ou (5) sur le dérivé de formule (6) on utilise généralement une quantité de catalyseur au platine calculée en poids de platine métal, comprise entre 5 et 600 ppm, de préférence entre 10 et 200 ppm, basée sur le poids de polymère à SiH de formule (4) ou (5).

La réaction d'hydrosilylation peut avoir lieu en masse ou au sein d'un solvant organique volatil tel que le toluène, l'heptane, le xylène, le tétrahydrofuranne et le tétrachloréthylène.

Il est généralement souhaitable de chauffer le mélange réactionnel à une température de 60 à 120°C pendant le temps nécessaire pour que la réaction soit complète. On peut ajouter goutte à goutte le polymère à SiH sur le dérivé de formule (6) en solution dans un solvant organique contenant le catalyseur. On peut aussi ajouter simultanément le polymère à SiH et le dérivé de formule (6) à une suspension de catalyseur (6) à une suspension de catalyseur dans un solvant organique.

On vérifie que la réaction est complète en dosant les SiH résiduels par la potasse alcoolique, puis on élimine le solvant, par exemple par distillation sous pression réduite.

L'huile brute obtenue peut être purifiée, par exemple par passage sur une colonne absorbante de silice.

Un autre objet de l'invention est constitué par les compositions cosmétiques destinées à protéger la peau et les cheveux du rayonnement UV, contenant une quantité efficace d'un diorganopolysiloxane à fonction dibenzoylméthane de formule (1) ou (2), dans un milieu cosmétiquement acceptable.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (1) ou (2) contenu dans un support cosmétiquement acceptable comprenant au moins une phase grasse.

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un diorganopolysiloxane à fonction dibenzoylméthane de formule (1) ou (2) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses, alcooliques ou oléoalcooliques, d'émulsions telles qu'une crème ou un lait, de gels oléoalcooliques, alcooliques ou hydroalcooliques, de bâtonnets solides ou être conditionnée en aérosol.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (1) ou (2) est présent dans des proportions en poids comprises entre 0,25 et 3% par rapport au poids total de la composition cosmétique protectrice de l'épiderme humain.

Comme solvant de solubilisation, on peut utiliser une huile, une cire et de façon générale tout corps gras, un monoalcool ou un polyol inférieur, un benzoate d'alcools en $C_{12}$-$C_{15}$ ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (1) ou (2), des alcools gras, des esters d'acides gras et notamment

des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques tels que les alcanes, les fluoroalcanes et les chlorofluoroalcanes.

La présente invention vise également les compositions cosmétiques anti-solaires contenant au moins un composé de formule (1) ou (2) et pouvant contenir d'autres filtres UV-B et/ou UV-A.

Dans ce cas, la quantité totale de filtres présents dans la composition anti-solaire, c'est-à-dire le composé de formule (1) ou (2) et éventuellement les autres filtres, est comprise entre 0,5 et 15% en poids par rapport au poids total de la composition anti-solaire.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de spray de coiffage, de laque pour cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 5% en poids de composé de formule (1) ou (2).

La présente invention vise également les compositions cosmétiques, contenant au moins un composé de formule (1) ou (2) à titre d'agent de protection contre les rayons ultraviolets, constituées par des compositions capillaires tels que les laques pour cheveux, les lotions de mise en plis éventuellement traitantes ou démêlantes, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,25 à 3% en poids de composé de formule (1) ou (2).

L'invention vise également un procédé de protection des compositions cosmétiques contre les rayons ultraviolets, consistant à incorporer à ces compositions une quantité efficace d'au moins un composé de formule (1) ou (2).

Les exemples ci-après illustrent l'invention sans en limiter la portée.

EXEMPLE 1

Préparation du polymère statistique de formule :

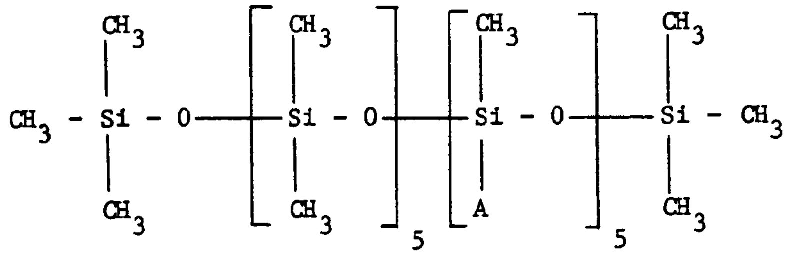 (1)

dans laquelle A est le reste de formule :

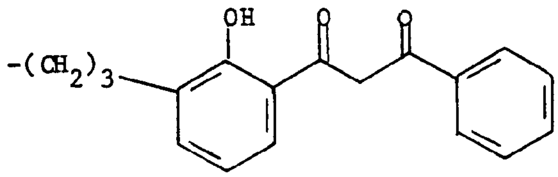

A une suspension de platine sur charbon à 5% (70 mg) dans du toluène sec (5 ml) à 90-100°C, sous azote et sous agitation, on ajoute goutte à goutte en 1 heure 30 minutes, une solution toluénique (40 ml) de 12,6 g (45 meq) d'allyl-3 hydroxy-2 dibenzoylméthane et 5,12 g de polyméthylhydrogénodiméthylsiloxane de formule ci-dessus où A est un atome d'hydrogène.

Tout en maintenant la température entre 100 et 105°C, on laisse sous agitation et au reflux jusqu'à disparition des groupements SiH (absence de bande à 2180 $cm^{-1}$ en infrarouge), soit 10 heures. On filtre sur papier, on élimine le solvant et on lave trois à l'éthanol à 80%. L'huile obtenue est reprise dans le chloroforme, séchée sur sufate de sodium et filtrée sur célite pour éliminer les restes de platine colloïdal. On obtient après évaporation du solvant une huile épaisse jaune orange (poids : 9,3 g, rendement : 68%).

Spectre UV ($CHCl_3$), $\lambda$ $max_1$= 346 nm,
$\lambda$ $max_2$= 365 nm,

L'analyse par résonance magnétique nucléaire ($^1H$ et $^{29}Si$ RMN) indique que ce produit est bien le polymère de formule ci-dessus.

## EXEMPLE 2

Préparation du polymère statistique de même formule qu'à l'exemple 1 sauf que A est un reste de formule :

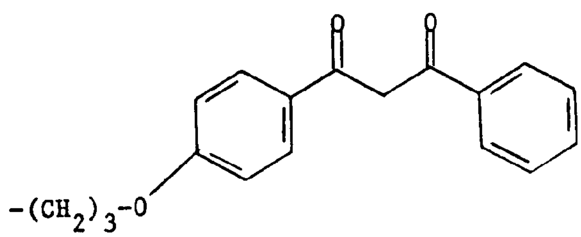

On applique le même mode opératoire que dans l'exemple 1 avec 10 g (36 meq) d'allyloxy-4 dibenzoylméthane et 5,8 g de polyméthylhydrogénodiméthylsiloxane. On obtient une huile épaisse jaune (poids : 15 g, rendement : 95%).

Spectre UV ($CHCl_3$) : $\lambda$ max = 353 nm

L'analyse par résonance magnétique nucléaire ($1_H$ et $^{29}Si$ RMN) indique que ce produit est bien un polymère de formule ci-dessus.

EXEMPLES D'APPLICATION

Exemple A : Emulsion huile-dans-l'eau (anti-solaire)

| | | |
|---|---|---|
| - Composé de l'exemple 2 | | 2,0 g |
| - Hydroxy-2 méthoxy-4 benzophénone | | 1,0 g |
| - Lanoline liquide | | 7,0 g |
| - Triglycérides d'acides myristique/palmitique/stéarique ("NESATOL" vendu par la Société VEVY) | | 5,0 g |
| - Triglycérides oléïques oxyéthylénés ("LUBRAFIL M1969 CS" vendu par la Société GATTEFOSSE) | | 2,5 g |
| - Mélange de monostéarate de glycérol et de stéarate de polyéthylène glycol (100 OE) ("ARLACEL 165" vendu par la Société SEPPIC) | | 5,0 g |
| - Alcool stéarylique | | 1,0 g |
| - Acide stéarique | | 2,5 g |
| - Mélange de phosphates de cétyle et de monocétyl phosphate de diéthanolamine ("AMPHISOL NP"vendu par la Société GIVAUDAN) | | 0,5 g |
| - Benzoate d'alcools C12/C15 ("FINSOLV TN" vendu par la Société WITCO) | | 9,0 g |
| - Triéthanolamine | | 0,2 g |
| - Conservateur | | 0,4 g |
| - Parfum | | 0,6 g |
| - Eau déminéralisée | qsp | 100 g |

Exemple B : Emulsion huile-dans-l'eau (crème protectrice de l'épiderme humain)

| | | |
|---|---|---|
| - Composé de l'exemple 1 | | 3,0 g |
| - Mélange d'alcools cétylstéarylique et cétyl stéarylique oxyéthyléné à 33 moles d'OE ("SINNOWAX AO" vendu par la Société HENKEL) | | 7,0 g |
| - Monostéarate de glycérol | | 2,0 g |
| - Alcool cétylique | | 1,3 g |
| - Propylène glycol | | 10,0 g |
| - Conservateur | | 0,2 g |
| - Parfum | | 0,6 g |
| - Benzoate d'alcools C12/C15 ("FINSOLV TN" vendu par la Société WITCO) | | 15,00 g |
| - Eau déminéralisée | qsp | 100 g |

Ces émulsions sont préparées en chauffant vers 80°-85°C les corps gras et les émulsionnants; à cette température on y ajoute le diorganopolysiloxane à fonction dibenzoylméthane. Par ailleurs, on chauffe à 80-85°C l'eau contenant les composés hydrosolubles et on ajoute la phase grasse à la phase aqueuse. Après 10 minutes d'agitation vive, on laisse refroidir sous agitation modérée et on ajoute le parfum et le conservateur vers 40°C.

**Revendications**

1. Utilisation en cosmétique de diorganopolysiloxanes à fonction dibenzoylméthane choisis parmi ceux de formule :

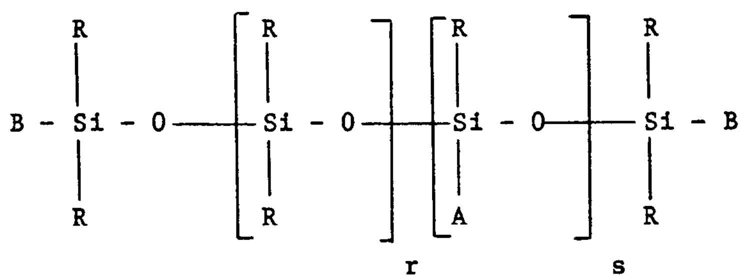

(1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

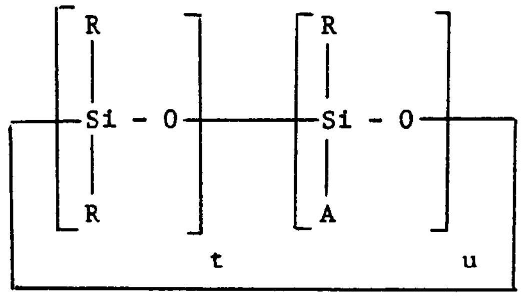

(2)

dans laquelle

R a la même signification qu'à la formule (1),

u est un nombre compris entre 1 et 20 inclus,

t est un nombre compris entre 0 et 20 inclus,

t + u est égal ou supérieur à 3,

formules dans lesquelles le symbole A est un radical de formule :

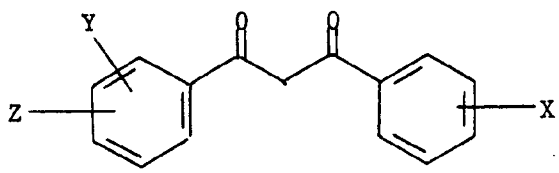

(3)

dans laquelle :

X est choisi parmi un atome d'hydrogène, un radical alkyle linéaire ou ramifié en $C_1$-$C_8$, un radical alcoxy linéaire ou ramifié en $C_1$-$C_8$,

Y est choisi parmi un atome d'hydrogène, un groupe hydroxyle et un radical alcoxy linéaire ou ramifié en $C_1$-$C_4$,

Z est un reste divalent de formule :

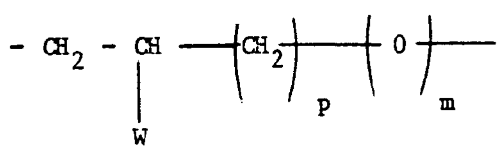

dans lequel m est 0 ou 1, p est un nombre entier compris entre 1 et 10 inclusivement et W est choisi parmi un atome d'hydrogène et un radical alkyle en $C_1$-$C_4$, sous réserve que, lorsque m est égal à 0, Y représente un radical alcoxy ou OH et soit situé en position ortho par rapport à Z.

**2.** Utilisation en cosmétique d'un polymère statistique ou à blocs de formule (1) ou (2) selon la revendication 1, présentant au moins l'une des caractéristiques suivantes :

R est méthyle,

B est méthyle,

r est compris entre 5 et 20 inclus,

s est compris entre 2 et 15 inclus,

t + u est compris entre 3 et 10 inclus,

X est H,

Y est H, OH ou méthoxy,

Z est un reste divalent dans lequel m = 0 ou 1, p = 1, W = H ou méthyle.

**3.** Utilisation en cosmétique à titre d'agents filtrant le rayonnement UV de longueurs d'onde comprises entre 280 et 360 nm, de diorganopolysiloxanes à fonction dibenzoylméthane choisis parmi ceux de formule :

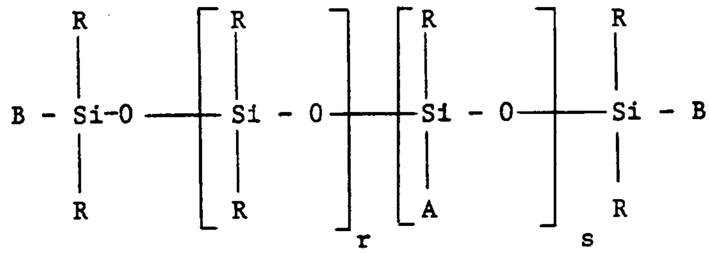

(1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

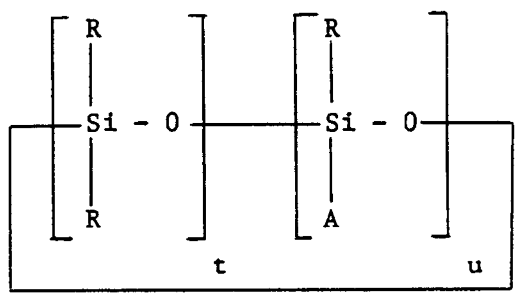

(2)

dans laquelle

R a la même signification qu'à la formule (1),

u est un nombre compris entre 1 et 20 inclus,

t est un nombre compris entre 0 et 20 inclus,

t + u est égal ou supérieur à 3,

formules dans lesquelles le symbole A est un radical de formule :

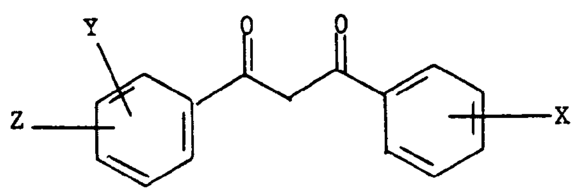

(3)

dans laquelle :

- X est choisi parmi un atome d'hydrogène, un radical alkyle linéaire ou ramifié en $C_1$-$C_8$, un radical alcoxy linéaire ou ramifié en $C_1$-$C_8$,
- Y est choisi parmi un atome d'hydrogène, un groupe hydroxyle et un radical alcoxy linéaire ou ramifié en $C_1$-$C_4$,
- Z est un reste divalent de formule :

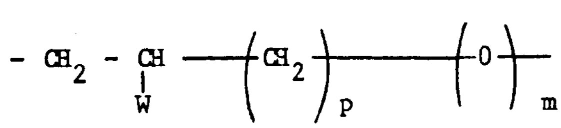

dans lequel m est 0 ou 1, p est un nombre entier compris entre 1 et 10 inclusivement et W est choisi parmi un atome d'hydrogène et un radical alkyle en $C_1$-$C_4$, sous réserve que, lorsque m est égal à 0, Y représente un radical alcoxy ou OH et soit situé en position ortho par rapport à Z.

**4.** Utilisation en cosmétique à titre d'agents filtrant le rayonnement UV de longueurs d'onde comprises entre 280 et 360 nm, de diorganopolysiloxanes statistiques ou à blocs de formule (1) ou (2) selon la revendication 3, présentant au moins l'une des caractéristiques suivantes :

R est méthyle
B est méthyle
r est compris entre 5 et 20 inclus,
s est compris entre 2 et 15 inclus,
t + u est compris entre 3 et 10 inclus,
X est H,
Y est H, OH ou méthoxy,
Z est un reste divalent dans lequel m = 0 ou 1, p = 1, W = H ou méthyle.

**5.** Utilisation en cosmétique à titre d'agent filtrant le rayonnement UV de longueurs d'onde comprises entre 280 et 360 nm, d'un polydiméthylsiloxane à greffons hydroxy-2-allyl-3 dibenzoylméthane de formule (1) selon la revendication 1 dans laquelle R et B désignent méthyle, r = 5 et s =5.

**6.** Utilisation en cosmétique à titre d'agent filtrant le rayonnement UV de longueurs d'onde comprises entre 280 et 360 nm, d'un polydiméthylsiloxane à greffons allyloxy-4 dibenzoylméthane de formule (1) selon la revendication 1, dans laquelle R et B désignent méthyle, r = 5 et s = 5.

**7.** Composition cosmétique, caractérisée par le fait qu'elle comprend, dans un support cosmétiquement acceptable, une quantité efficace d'au moins un diorganopolysiloxane à fonction dibenzoylméthane choisi parmi ceux de formule :

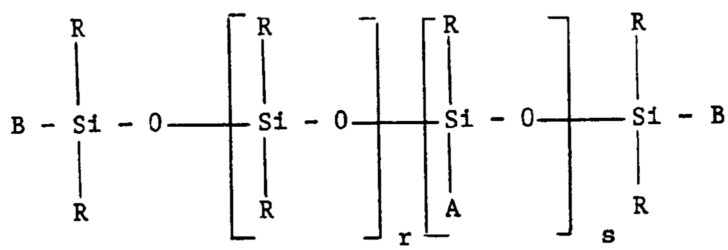

(1)

dans laquelle les symboles :

R, identiques ou différents, sont choisis parmi les radicaux alkyle linéaires ou ramifiés en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre choisi entre 0 et 200 inclusivement,

s est un nombre choisi entre 0 et 50 inclusivement et si s est 0, au moins l'un des deux symboles B désigne A,

et ceux de formule :

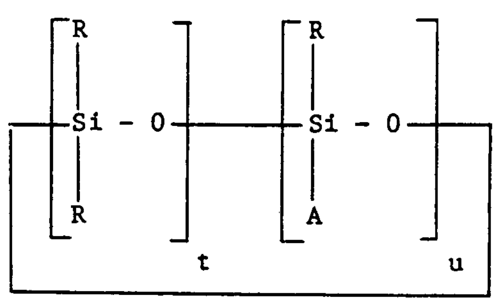

(2)

dans laquelle

R a la même signification qu'à la formule (1),
u est un nombre compris entre 1 et 20 inclus,
t est un nombre compris entre 0 et 20 inclus,
t + u est égal ou supérieur à 3,

formules dans lesquelles le symbole A est un radical de formule :

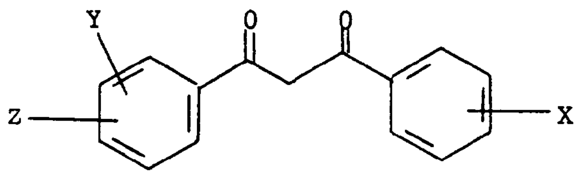

(3)

dans laquelle :

- X est choisi parmi un atome d'hydrogène, un radical alkyle linéaire ou ramifié en $C_1$-$C_8$, un radical alcoxy linéaire ou ramifié en $C_1$-$C_8$,
- Y est choisi parmi un atome d'hydrogène, un groupe hydroxyle et un radical alcoxy linéaire ou ramifié en $C_1$-$C_4$,
- Z est un reste divalent de formule

$$-CH_2-\underset{\displaystyle W}{\underset{|}{CH}}-\left(CH_2\right)_p-\left(O\right)_m-$$

dans lequel m est 0 ou 1, p est un nombre entier compris entre 1 et 10 inclusivement et W est choisi parmi un atome d'hydrogène et un radical alkyle en $C_1$-$C_4$, sous réserve que, lorsque m est égal à 0, Y représente un radical alcoxy ou OH et soit situé en position ortho par rapport à Z.

**8.** Composition cosmétique selon la revendication 7, caractérisée par le fait qu'elle comprend un diorganopolysiloxane à fonction dibenzoylméthane de formule (1) ou (2), statistique ou à blocs, présentant au moins l'une des caractéristiques suivantes : R est méthyle, B est méthyle, r est compris entre 5 et 20 inclus, s est compris entre 2 et 15 inclus, t + u est compris entre 3 et 10 inclus, X est H, Y est H, OH ou méthoxy, Z est un reste divalent dans lequel m = 0 ou 1, p = 1, W = H ou méthyle.

**9.** Composition cosmétique selon la revendication 7 ou 8, caractérisée par le fait qu'elle comprend un polydiméthylsiloxane à greffons hydroxy-2-allyl-3 dibenzoylméthane de formule (1) selon la revendication 7 dans laquelle R et B désignent méthyle, r = 5, s = 5.

**10.** Composition cosmétique selon la revendication 7 ou 8, caractérisée par le fait qu'elle comprend un polydiméthylsiloxane à greffons allyloxy-4 dibenzoylméthane de formule (1) dans laquelle R et B désignent méthyle, r = 5 et s = 5.

**11.** Composition cosmétique selon l'une quelconque des revendications 7 à 10, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, benzoates d'alcools en $C_{12}$-$C_{15}$, propulseurs, colorants et pigments.

**12.** Composition cosmétique selon l'une quelconque des revendications 7 à 11, caractérisée par le fait qu'elle se présente sous forme de lotion huileuse, alcoolique ou oléoalcoolique, d'émulsion, gel oléoalcoolique, alcoolique ou hydroalcoolique, bâtonnet solide, spray ou aérosol.

**13.** Composition cosmétique selon l'une quelconque des revendications 7 à 12, caractérisée par le fait qu'elle constitue une composition protectrice de l'épiderme humain et contient 0,25 à 3% en poids de diorganopolysiloxane de formule (1) ou (2).

**14.** Composition cosmétique selon l'une quelconque des revendications 7 à 12, se présentant sous forme de composition anti-solaire, caractérisée par le fait qu'elle contient 0,5 à 15% en poids de diorganopolysiloxane de formule (1) ou (2).

**15.** Composition cosmétique anti-solaire selon la revendication 14, caractérisée par le fait qu'elle contient en outre un agent filtrant les rayons UV-B et/ou UV-A.

**16.** Composition cosmétique selon l'une quelconque des revendications 7 à 11, destinée à être appliquée sur les cheveux, caractérisée par le fait qu'elle se présente sous forme de shampooing, lotion, gel ou émulsion à rincer, à appliquer avant ou après le shampooing, avant ou après coloration ou décoloration, avant ou après permanente, lotion ou gel coiffants ou traitants, lotion ou gel pour le brushing ou la mise en plis, spray de coiffage, laque pour cheveux, et comprend 0,25 à 5% en poids de diorganopolysiloxane de formule (1) ou (2).

**17.** Composition cosmétique selon l'une quelconque des revendications 7 à 11, se présentant sous forme d'une composition cosmétique colorée ou non, caractérisée par le fait qu'elle est constituée par une composition capillaire, un produit de maquillage ou une composition pour les soins ou le traitement de la peau, comprenant 0,25 à 3% en poids de diorganopolysiloxane de formule (1) ou (2).

**18.** Procédé de protection de la peau et des cheveux naturels ou sensibilisés contre le rayonnement ultraviolet, caractérisé par le fait qu'il consiste à appliquer sur la peau ou les cheveux une quantité efficace d'une composition cosmétique contenant au moins un diorganopolysiloxane à fonction dibenzoylméthane de formule (1) ou (2) définie dans l'une quelconque des revendications 3 à 6.

**19.** Procédé de protection d'une composition cosmétique contre les rayons ultraviolets, caractérisé par le fait qu'il consiste à incorporer à cette composition une quantité efficace d'au moins un diorganopolysiloxane à fonction dibenzoylméthane de formule (1) ou (2) définie dans l'une quelconque des revendications 3 à 6.

**Patentansprüche**

**1.** Verwendung in der Kosmetik von Diorganopolysiloxanen mit Dibenzoylmethan-Funktion, ausgewählt unter denen der Formel:

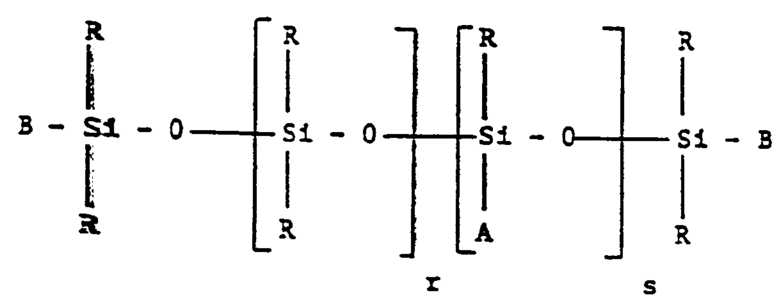

(1)

worin die Symbole das folgende bedeuten:
die Reste R, gleich oder verschieden, sind ausgewählt unter linearen oder verzweigten $C_{1-10}$-Niedrigalkyl-, Phenyl- und 3,3,3-Trifluorpropylresten, wobei mindestens 80% der Anzahl der Reste R Methylreste sind,
die Reste B, gleich oder verschieden, sind ausgewählt unter den Resten R und dem Rest A,
r ist eine Zahl von 0 bis 200,
s ist eine Zahl von 0 bis 50, und wenn s 0 ist, bedeutet mindestens eines der beiden Symbole B A;
sowie ausgewählt unter denen der Formel:

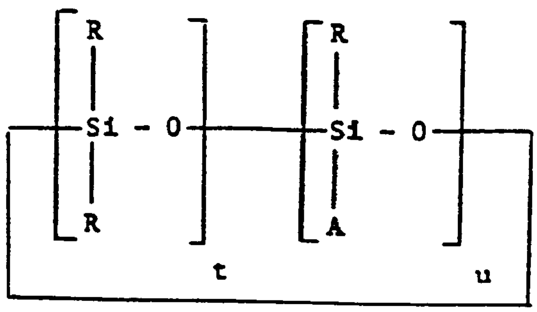

(2)

worin
R dieselbe Bedeutung wie in Formel (1) hat,
u eine Zahl von 1 bis 20 ist,
t eine Zahl von 0 bis 20 ist,
t+u gleich oder größer 3 ist,
wobei in den Formeln das Symbol A ein Rest der Formel ist:

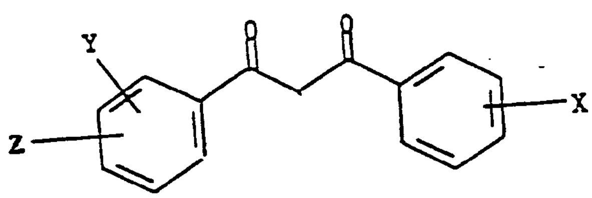

(3)

worin:
X unter einem Wasserstoffatom, einem linearen oder verzweigten $C_1$-$C_8$-Alkylrest und einem linearen oder verzweigten $C_1$-$C_8$-Alkoxyrest ausgewählt ist,
Y unter einem Wasserstoffatom, einer Hydroxylgruppe und einem linearen oder verzweigten $C_1$-$C_4$-Alkoxyrest ausgewählt ist,
Z ein zweiwertiger Rest der Formel ist:

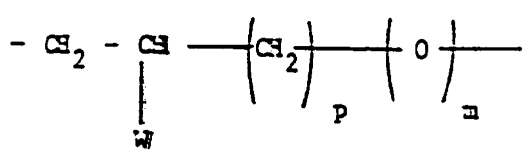

worin m 0 oder 1 und p eine ganze Zahl von 1 bis 10 sind und W unter einem Wasserstoffatom und einem $C_{1-4}$-Alkylrest ausgewählt ist, unter der Maßgabe, daß, wenn m gleich 0 ist, Y einen Alkoxyrest oder OH darstellt und sich in ortho-Position zu Z befindet.

2. Verwendung in der Kosmetik gemäß Anspruch 1 eines statistischen Polymers oder eines Blockpolymers der Formel (1) oder (2), wobei die Formeln mindestens eines der folgenden Merkmale aufweisen:
R ist ein Methylrest,
B ist ein Methylrest,
r beträgt 5 bis 20,
s beträgt 2 bis 15,
t + u beträgt 3 bis 10,
X ist H,
Y ist H, OH oder ein Methoxyrest,
Z ist ein zweiwertiger Rest, worin m = 0 oder 1, p = 1, W = H oder ein Methylrest.

3. Verwendung von Diorganospolysiloxanen mit Dibenzoylmethan-Funktion in der Kosmetik als Mittel, die die UV-Strahlung von Wellenlängen von 280 bis 360 nm filtern, wobei die Diorganopolysiloxane ausgewählt sind unter denen der Formel:

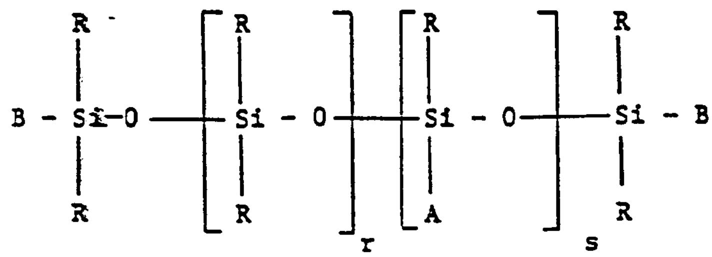

(1)

worin die Symbole das folgende bedeuten:
die Reste R, gleich oder verschieden, sind ausgewählt unter linearen oder verzweigten $C_{1-10}$-Niedrigalkyl-, Phenyl- und 3,3,3-Trifluorpropylresten, wobei mindestens 80% der Anzahl der Reste R Methylreste sind,
die Reste B, gleich oder verschieden, sind ausgewählt unter den Resten R und dem Rest A,
r ist eine Zahl von 0 bis 200,
s ist eine Zahl von 0 bis 50, und wenn s 0 ist, bedeutet mindestens eines der beiden Symbole B A;
sowie ausgewählt unter denen der Formel:

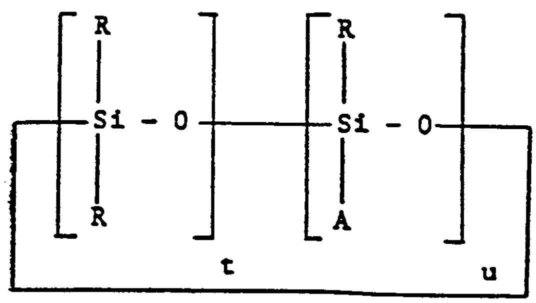

(2)

worin
R dieselbe Bedeutung wie in Formel (1) hat,
u eine Zahl von 1 bis 20 ist,
t eine Zahl von 0 bis 20 ist,
t+u gleich oder größer 3 ist,
wobei in den Formeln das Symbol A ein Rest der Formel ist:

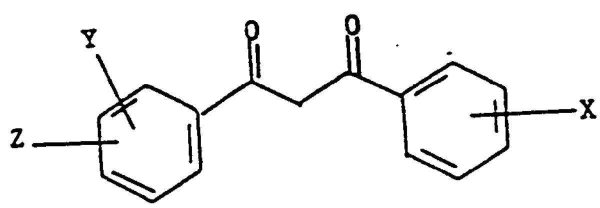

(3)

worin:
X unter einem Wasserstoffatom, einem linearen oder verzweigten $C_1$-$C_8$-Alkylrest und einem linearen oder verzweigten $C_1$-$C_8$-Alkoxyrest ausgewählt ist,
Y unter einem Wasserstoffatom, einer Hydroxylgruppe und einem linearen oder verzweigten $C_1$-$C_4$-Alkoxyrest ausgewählt ist,
Z ein zweiwertiger Rest der Formel ist:

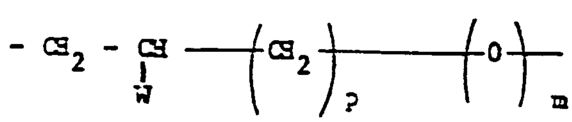

worin m 0 oder 1 und p eine ganze Zahl von 1 bis 10 sind und W unter einem Wasserstoffatom und einem

$C_{1-4}$-Alkylrest ausgewählt ist, unter der Maßgabe, daß, wenn m gleich 0 ist, Y einen Alkoxyrest oder OH darstellt und sich in ortho-position zu Z befindet.

4. Verwendung in der Kosmetik gemäß Anspruch 3 von statistischen oder Blockdiorganopolyisiloxanen der Formel (1) oder (2) als Mittel, die die UV-Strahlung von Wellenlängen von 280 bis 360 nm filtern, wobei die Formeln mindestens eines der folgenden Merkmale aufweisen:
R ist ein Methylrest,
B ist ein Methylrest,
r beträgt 5 bis 20,
s beträgt 2 bis 15,
t + u beträgt 3 bis 10,
X ist H,
Y ist H, OH oder ein Methoxyrest,
Z ist ein zweiwertiger Rest, worin m = 0 oder 1, p = 1, W = H oder ein Methylrest.

5. Verwendung gemäß Anspruch 1 eines Polydimethylsiloxans, gepfropft mit 2-Hydroxy-3-allyldibenzoylmethan, der Formel (1), worin R und B Methylreste darstellen, r = 5 und s = 5, als Mittel, das die UV-Strahlung von Wellenlängen von 280 bis 360 nm filtert.

6. Verwendung gemäß Anspruch 1 eines Polydimethylsiloxans, gepfropft mit 4-Allyloxydibenzoylmethan, der Formel (1), worin R und B Methylreste bedeuten, r = 5 und s = 5, als Mittel, das die UV-Strahlung von Wellenlängen von 280 bis 360 nm filtert.

7. Kosmetische Zusammensetzung,
dadurch **gekennzeichnet**, daß
sie, in einem kosmetisch verträglichen Träger, eine wirksame Menge mindestens eines Dioganopolysiloxans mit Dibenzoylmethan-Funktion enthält, ausgewählt unter denen der Formel:

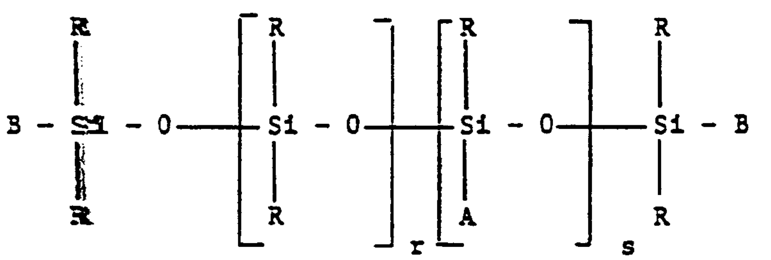

(1)

worin die Symbole das folgende bedeuten:
die Reste R, gleich oder verschieden, sind ausgewählt unter linearen oder verzweigten $C_{1-10}$-Niedrigalkyl-, Phenyl- und 3,3,3-Trifluorpropylresten, wobei mindestens 80% der Anzahl der Reste R Methylreste sind,
die Reste B, gleich oder verschieden, sind ausgewählt unter den Resten R und dem Rest A,
r ist eine Zahl von 0 bis 200,
s ist eine Zahl von 0 bis 50, und wenn s 0 ist, bedeutet mindestens eines der beiden Symbole B A;
sowie ausgewählt unter denen der Formel:

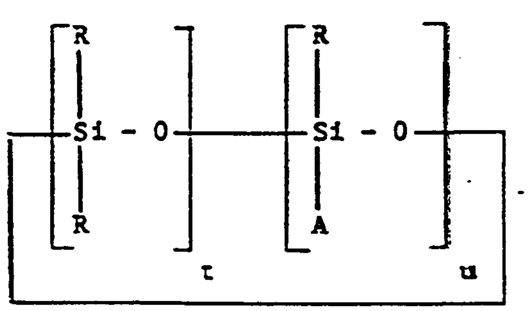

(2)

worin

R dieselbe Bedeutung wie in Formel (1) hat,
u eine Zahl von 1 bis 20 ist,
t eine Zahl von 0 bis 20 ist,
t+u gleich oder größer 3 ist,
wobei in den Formeln das Symbol A ein Rest der Formel ist:

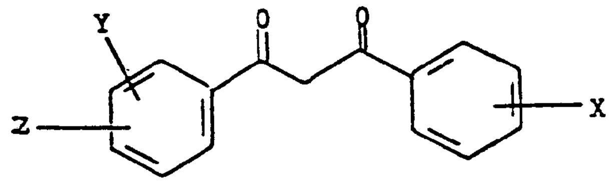

(3)

worin:
X unter einem Wasserstoffatom, einem linearen oder verzweigten $C_1$-$C_8$-Alkylrest und einem linearen oder verzweigten $C_1$-$C_8$-Alkoxyrest ausgewählt ist,
Y unter einem Wasserstoffatom, einer Hydroxylgruppe und einem linearen oder verzweigten $C_1$-$C_4$-Alkoxyrest ausgewählt ist,
Z ein zweiwertiger Rest der Formel ist:

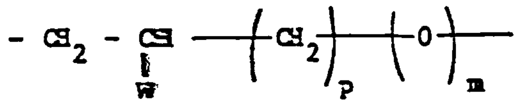

worin m 0 oder 1 und p eine ganze Zahl von 1 bis 10 sind und W unter einem Wasserstoffatom und einem $C_{1-4}$-Alkylrest ausgewählt ist, unter der Maßgabe, daß, wenn m gleich 0 ist, Y einen Alkoxyrest oder OH darstellt und sich in ortho-Position zu Z befindet.

**8.** Kosmetische Zusammensetzung nach Anspruch 7,
dadurch **gekennzeichnet**, daß
sie ein statistisches oder Blockdiorganopolysiloxan mit Dibenzoylmethan-Funktion der Formel (1) oder (2) enthält, die mindestens eines der folgenden Merkmale aufweisen: R ist ein Methylrest, B ist ein Methylrest, r beträgt 5 bis 20, s beträgt 2 bis 15, t + u beträgt 3 bis 10, X ist H, Y ist H, OH oder ein Methoxyrest, Z ist ein zweiwertiger Rest, worin m = 0 oder 1, p = 1, W = H oder ein Methylrest.

**9.** Kosmetische Zusammensetzung gemäß Anspruch 7 oder 8,
dadurch **gekennzeichnet**, daß
sie ein mit 2-Hydroxy-3-allyldibenzoylmethan gepfropftes Polydimethylsiloxan der Formel (1) gemäß Anspruch 7 enthält, worin R und B Methylreste bedeuten, r = 5, s = 5.

**10.** Kosmetische Zusammensetzung gemäß Anspruch 7 oder 8,
dadurch **gekennzeichnet**, daß
sie ein mit 4-Allyloxydibenzoylmethan gepfropftes Polydimethylsiloxan der Formel (1) enthält, worin R und B Methylreste bedeuten, r = 5 und s = 5.

**11.** Kosmetische Zusammensetzung gemäß jedem der Ansprüche 7 bis 10,
dadurch **gekennzeichnet**, daß
sie zusätzlich kosmetische Hilfsstoffe enthält, ausgewählt unter Verdickungsmitteln, Enthärtungsmitteln, Feuchtigkeitsmitteln, oberflächenaktiven Mitteln, Konservierungsmitteln, Antischaummitteln, Parfums, Ölen, Wachsen, Lanolin, Niedrigmonoalkoholen und -polyolen, $C_{12-15}$-Alkylbenzoaten, , Treibmitteln, Färbemitteln und Pigmenten.

**12.** Kosmetische Zusammensetzung gemäß jedem der Ansprüche 7 bis 11,
dadurch **gekennzeichnet**, daß
sie in Form einer öligen, alkoholischen oder oleoalkoholischen Lotion, einer Emulsion, eines oleoalkoholischen, alkoholischen oder hydroalkoholischen Gels, eines Feststoffstäbchens, Sprays oder Aerosols vorliegt.

13. Kosmetische Zusammensetzung gemäß jedem der Ansprüche 7 bis 12,
dadurch **gekennzeichnet**, daß
sie eine die Haut schützende Zusammensetzung darstellt und 0,25 bis 3 Gew.% Diorganopolysiloxan der Formel (1) oder (2) enthält.

14. Kosmetische Zusammensetzung gemäß jedem der Ansprüche 7 bis 12,
die in Form einer Zusammensetzung zum Schutz vor Sonneneinwirkung vorliegt,
dadurch **gekennzeichnet**, daß
sie 0,5 bis 15 Gew.% Diorganopolysiloxan der Formel (1) oder (2) enthält.

15. Kosmetische Zusammensetzung zum Schutz vor Sonneneinwirkung gemäß Anspruch 14,
dadurch **gekennzeichnet**, daß
sie zusätzlich ein weiteres Mittel enthält, das die Strahlen UV-B und/oder UV-A filtert.

16. Kosmetische Zusammensetzung gemäß jedem der Anprüche 7 bis 11 zur Aufbringung auf die Haare,
dadurch **gekennzeichnet**, daß
sie in Form eines Shampoos, einer Lotion, eines Gels oder einer Emulsion zur Spülung, zur Aufbringung vor oder nach dem Shampoonieren, vor oder nach der Färbung oder Entfärbung, vor oder nach der Dauerwelle, einer Frisur- oder Behandlungslotion oder eines entsprechenden Gels, einer Lotion oder eines Gels zum Bürsten oder zur Wellengebung, eines Frisursprays oder eines Lackes für die Haare vorliegt und 0,25 bis 5 Gew.% Diorganopolysiloxan der Formel (1) oder (2) enthält.

17. Kosmetische Zusammensetzung gemäß jedem der Ansprüche 7 bis 11, die in Form einer gefärbten oder nicht gefärbten kosmetischen Zusammensetzung vorliegt,
dadurch **gekennzeichnet**, daß
sie eine Zusammensetzung für die Haare, ein Schminkprodukt oder eine Zusammensetzung zur pflege oder Behandlung der Haut darstellt und 0,25 bis 3 Gew.% Diorganopolysiloxan der Formel (1) oder (2) enthält.

18. Verfahren zum Schutz der Haut und der natürlichen oder sensibilisierten Haare vor ultravioletter Strahlung,
dadurch **gekennzeichnet**, daß
man auf die Haut oder die Haare eine wirksame Menge einer kosmetischen Zusammensetzung aufbringt, enthaltend mindestens ein Diorganopolysiloxan mit Dibenzoylmethan-Funktion der Formel (1) oder (2), definiert in jedem der Ansprüche 3 bis 6.

19. Verfahren zum Schutz einer kosmetischen Zusammensetzung vor ultravioletten Strahlen,
dadurch **gekennzeichnet**, daß
man in diese Zusammensetzung eine wirksame Menge mindestens eines Diorganopolysiloxans mit Dibenzoylmethan-Funktion der Formel (1) oder (2), definiert in jedem der Ansprüche 3 bis 6, einbringt.

## Claims

1. Use in cosmetics of diorganopolysiloxanes, containing a dibenzoylmethane functional group, chosen from those of formula:

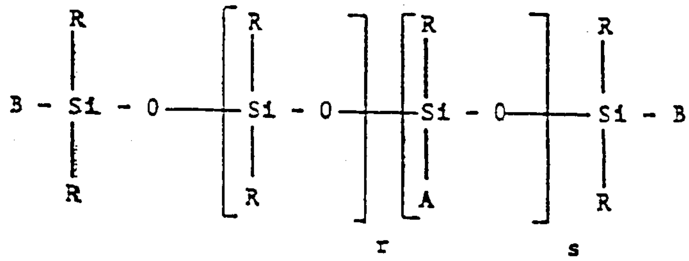

(1)

in which the symbols:

R, which are identical or different, are chosen from linear or branched $C_1$-$C_{10}$ alkyl radicals, phenyl radicals and from 3,3,3-trifluoropropyl radicals, at least 80% in numerical terms of the R radicals being methyl radicals,

B, which are identical or different, are chosen from the R radicals and the A radical,

r is a number chosen between 0 and 200 inclusive,

s is a number chosen between 0 and 50 inclusive, and, if s is 0, at least one of the two B symbols denotes A,

and those of formula:

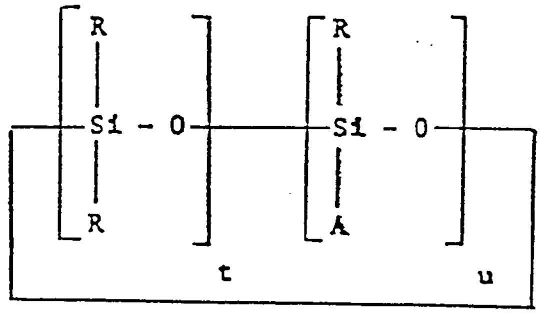

(2)

in which

R has the same meaning as in formula (1),

u is a number between 1 and 20 inclusive,

t is a number between 0 and 20 inclusive,

t + u is equal to or greater than 3,

formulae in which the symbol A is a radical of formula:

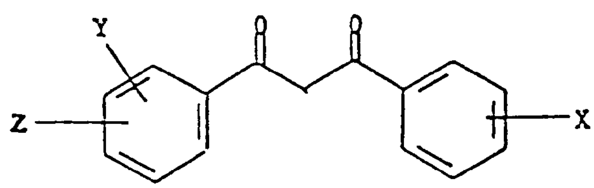

(3)

in which:

X is chosen from a hydrogen atom, a linear or branched $C_1$-$C_8$ alkyl radical and a linear or branched $C_1$-$C_8$ alkoxy radical,

Y is chosen from a hydrogen atom, a hydroxyl group and a linear or branched $C_1$-$C_4$ alkoxy radical,

Z is a divalent residue of formula:

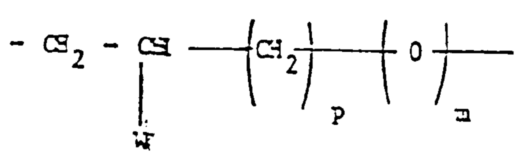

in which m is 0 or 1, p is an integer between 1 and 10 inclusive and W is chosen from a hydrogen atom and a $C_1$-$C_4$ alkyl radical, provided that, when m is equal to 0, Y represents an alkoxy or OH radical and is situated in the ortho position with respect to Z.

**2.** Use in cosmetics of a random or block polymer of formula (1) or (2) according to Claim 1, having at least one of the following characteristics:

R is methyl,

B is methyl,

r is between 5 and 20 inclusive,

s is between 2 and 15 inclusive,
t + u is between 3 and 10 inclusive,
X is H,
Y is H, OH or methoxy,
Z is a divalent residue in which m = 0 or 1, p = 1, W = H or methyl.

3. Use in cosmetics as agents screening out UV radiation of wavelengths between 280 and 360 nm, of diorganopolysiloxanes, containing a dibenzoylmethane functional group, chosen from those of formula:

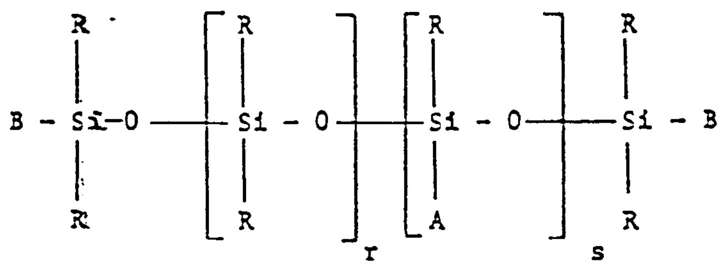

(1)

in which the symbols:

R, which are identical or different, are chosen from linear or branched $C_1$-$C_{10}$ alkyl radicals, phenyl radicals and from 3,3,3-trifluoropropyl radicals, at least 80% in numerical terms of the R radicals being methyl radicals,
B, which are identical or different, are chosen from the R radicals and the A radical,
r is a number chosen between 0 and 200 inclusive,
s is a number chosen between 0 and 50 inclusive, and, if s is 0, at least one of the two B symbols denotes A,

and those of formula:

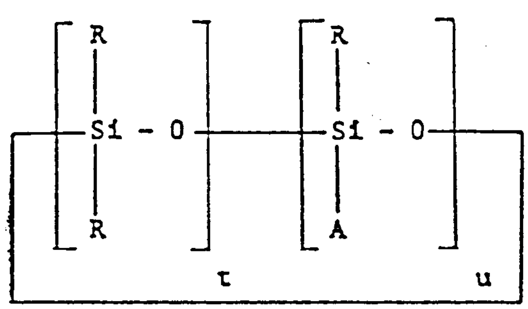

(2)

in which

R has the same meaning as in formula (1),
u is a number between 1 and 20 inclusive,
t is a number between 0 and 20 inclusive,
t + u is equal to or greater than 3,
formulae in which the symbol A is a radical of formula:

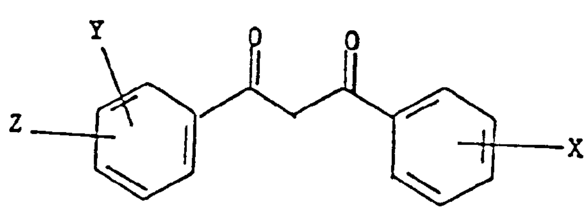

(3)

in which:

X is chosen from a hydrogen atom, a linear or branched $C_1$-$C_8$ alkyl radical and a linear or branched $C_1$-$C_8$ alkoxy radical,

Y is chosen from a hydrogen atom, a hydroxyl group and a linear or branched $C_1$-$C_4$ alkoxy radical,

Z is a divalent residue of formula:

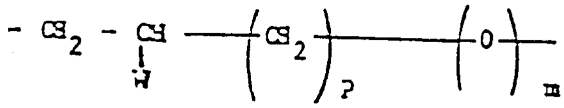

in which m is 0 or 1, p is an integer between 1 and 10 inclusive and W is chosen from a hydrogen atom and a $C_1$-$C_4$ alkyl radical, provided that, when m is equal to 0, Y represents an alkoxy or OH radical and is situated in the ortho position with respect to Z.

**4.** Use in cosmetics as agents screening out UV radiation of wavelengths between 280 and 360 nm, of random or block diorganopolysiloxanes of formula (1) or (2) according to Claim 3, having at least one of the following characteristics:

R is methyl,
B is methyl,
r is between 5 and 20 inclusive,
s is between 2 and 15 inclusive,
t + u is between 3 and 10 inclusive,
X is H,
Y is H, OH or methoxy,
Z is a divalent residue in which m = 0 or 1, p = 1, W = H or methyl.

**5.** Use in cosmetics as an agent for screening out UV radiation of wavelengths between 280 and 360 nm, of a polydimethylsiloxane containing 2-hydroxy-3-allyldibenzoylmethane grafts of formula (1) according to Claim 1, in which R and B denote methyl, r = 5 and s = 5.

**6.** Use in cosmetics as an agent for screening out UV radiation of wavelengths between 280 and 360 nm, of a polydimethylsiloxane containing 4-allyloxydibenzoylmethane grafts of formula (1) according to Claim 1, in which R and B denote methyl, r = 5 and s = 5.

**7.** Cosmetic composition, characterised in that it comprises, in a cosmetically acceptable carrier, an effective amount of at least one diorganopolysiloxane, containing a dibenzoylmethane functional group, chosen from those of formula:

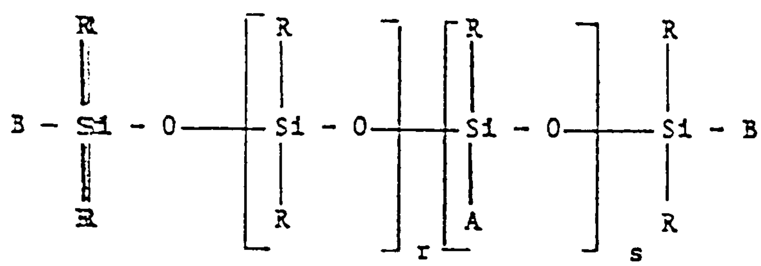

(1)

in which the symbols:

R, which are identical or different, are chosen from linear or branched $C_1$-$C_{10}$ alkyl radicals, phenyl radicals and from 3,3,3-trifluoropropyl radicals, at least 80% in numerical terms of the R radicals being methyl radicals,

B, which are identical or different, are chosen from the R radicals and the A radical,

r is a number chosen between 0 and 200 inclusive,

s is a number chosen between 0 and 50 inclusive, and, if s is 0, at least one of the two B symbols denotes A,

and those of formula:

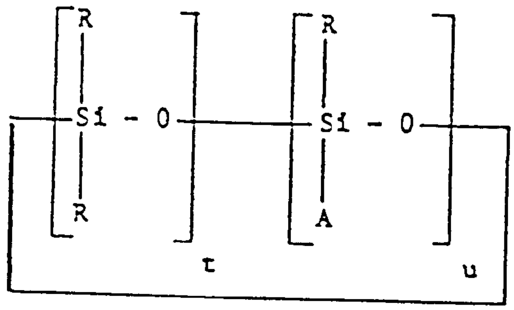

(2)

in which

R has the same meaning as in formula (1),
u is a number between 1 and 20 inclusive,
t is a number between 0 and 20 inclusive,
t + u is equal to or greater than 3,
formulae in which the symbol A is a radical of formula:

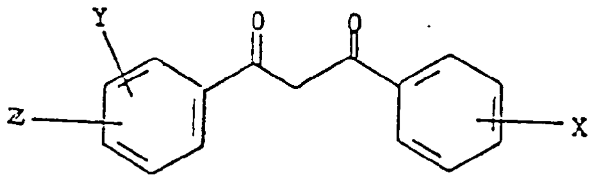

(3)

in which:

X is chosen from a hydrogen atom, a linear or branched $C_1$-$C_8$ alkyl radical and a linear or branched $C_1$-$C_8$ alkoxy radical,
Y is chosen from a hydrogen atom, a hydroxyl group and a linear or branched $C_1$-$C_4$ alkoxy radical,
Z is a divalent residue of formula:

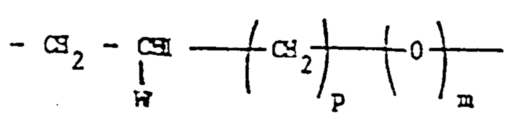

in which m is 0 or 1, p is an integer between 1 and 10 inclusive and W is chosen from a hydrogen atom and a $C_1$-$C_4$ alkyl radical, provided that, when m is equal to 0, Y represents an alkoxy or OH radical and is situated in the ortho position with respect to Z.

8. Cosmetic composition according to Claim 7, characterised in that it comprises a random or block diorganopolysiloxane, containing a dibenzoylmethane functional group of formula (1) or (2), having at least one of the following characteristics: R is methyl, B is methyl, r is between 5 and 20 inclusive, s is between 2 and 15 inclusive, t + u is between 3 and 10 inclusive, X is H, Y is H, OH or methoxy, Z is a divalent residue in which m = 0 or 1, p = 1, W = H or methyl.

9. Cosmetic composition according to Claim 7 or 8, characterised in that it comprises a polydimethylsiloxane containing 2-hydroxy-3-allyldibenzoylmethane grafts of formula (1) according to Claim 7, in which R and B denote methyl, r = 5, s = 5.

10. Cosmetic composition according to Claim 7 or 8, characterised in that it comprises a polydimethylsiloxane containing 4-allyloxydibenzoylmethane grafts of formula (1), in which R and B denote methyl, r = 5 and s = 5.

11. Cosmetic composition according to any one of Claims 7 to 10, characterised in that it also contains cosmetic adjuvants chosen from thickeners, emollients, humectants, surfactants, preservatives, antifoams,

perfumes, oils, waxes, lanolin, lower monoalcohols and polyols, $C_{12}$-$C_{15}$ alcohol benzoates, propellants, colorants and pigments.

12. Cosmetic composition according to any one of Claims 7 to 11, characterised in that it is provided in the form of an oily, alcoholic or oil-alcohol lotion, an emulsion, an oil-alcohol, alcoholic or water-alcohol gel, a solid stick, a spray or aerosol.

13. Cosmetic composition according to any one of Claims 7 to 12, characterised in that it constitutes a protective composition for the human epidermis and contains 0.25 to 3 % by weight of diorganopolysiloxane of formula (1) or (2).

14. Cosmetic composition according to any one of Claims 7 to 12, which is provided in the form of anti-sun composition, characterised in that it contains 0.5 to 15 % by weight of diorganopolysiloxane of formula (1) or (2).

15. Anti-sun cosmetic composition according to Claim 14, characterised in that it also contains an agent screening out UV-B and/or UV-A rays.

16. Cosmetic composition according to any one of Claims 7 to 11, which is intended to be applied to the hair, characterised in that it is provided in the form of a rinse-off shampoo, lotion, gel or emulsion to be applied before or after shampooing, before or after dyeing or bleaching, or before or after permanent waving, a hair styling or treating lotion or gel, a lotion or gel for blow drying or hair setting, a hair styling spray or a hair lacquer, and comprises 0.25 to 5 % by weight of diorganopolysiloxane of formula (1) or (2).

17. Cosmetic composition according to any one of Claims 7 to 11, which is provided in the form of a coloured or colourless cosmetic composition, characterised in that it consists of a hair composition, a make-up product or a composition for the care or treatment of the skin, comprising 0.25 to 3 % by weight of diorganopolysiloxane of formula (1) or (2).

18. Process for protecting the skin and natural or sensitised hair against ultraviolet radiation, characterised in that it consists in applying to the skin or hair an effective amount of cosmetic composition containing at least one diorganopolysiloxane containing a dibenzoylmethane functional group of formula (1) or (2) defined in any one of Claims 3 to 6.

19. Process for protecting a cosmetic composition against ultraviolet rays, characterised in that it consists in incorporating into this composition an effective amount of at least one diorganopolysiloxane containing a dibenzoylmethane functional group of formula (1) or (2) defined in any one of Claims 3 to 6.